# EUROPEAN PATENT SPECIFICATION

(11) **EP 1 830 761 B1**
(45) Date of publication and mention of the grant of the patent: **30.07.2014**
(21) Application number: 04809206.8
(22) Date of filing: 29.12.2004
(51) Int. Cl.: A61F 13/64, A61F 13/56, A61F 13/15

(54) **FASTENING MEANS IN THE FORM OF A BELT FOR AN ABSORBENT ARTICLE**
BEFESTIGUNGSMITTEL IN FORM EINES GÜRTELS FÜR EINEN SAUGFÄHIGEN ARTIKEL
MOYEN D'ATTACHE EN FORME DE CEINTURE POUR ARTICLE ABSORBANT

(43) Date of publication of application: 12.09.2007
(73) Proprietor: SCA Hygiene Products AB, 405 03 Göteborg (SE)
(72) Inventor: VARTIAINEN, Kent, 443 92 Lerum (SE)
(74) Representative: Valea AB
(86) International application number: PCT/SE2004/002033
(87) International publication number: WO 2006/071149

(56) References cited:
- WO-A1-02/22061
- US-A- 5 690 627
- US-A- 6 163 943
- US-A1- 2002 177 829

## Description

### Technical field

The present invention relates to a fastening means for an absorbent article in the form of a belt, which comprises at least one belt portion. The belt portion comprises at least one non-woven layer of composite material.

### Background

Fastening means such as belts give a user of an absorbent article good potential for adjustment of the article, both during and after application. Fastening takes place in two steps. The first step is when the belt is fastened about the wearer's waist, the second when the absorbent member is fastened to the belt, so that the article takes a pant-like form. Belt-diapers also provide carers, such as hospital personnel, the potential to minimise strained movements while applying the absorbent article.

However, a series of disadvantages exist with belt-diapers, and particularly with the belts. The belt is most often manufactured with a laminate structure, to provide a sufficient tensile strength and to give the belt sufficient stiffness. The laminate structure is most often produced from nonwoven material which had been ultrasonically welded, thermo-bonded or glued together. The manufacturing process is complicated and requires a plurality of manufacturing steps, and is also costly.

Known laminate belts are made of synthetic fibres. This implies that the belts are most-commonly hydrophobic. Sweating often occurs when a user lies in a bed, e.g. at night-time. It has been shown that known laminate belts cause uncomfortable sensations, as sweat can gather as a thin layer between the users trunk and the belt of the diaper. This becomes especially marked when the bonding area in a laminate belt is high. Under e.g. ultrasound welding, the bonding points have a structure such that it is difficult for liquid to penetrate the belt at these points. The problem becomes even more noticeable when there are a number of bonding points which are relatively small, but tightly spaced. Small amounts of moisture can be formed over a large surface between the belt and the skin of the user, leading to the skin being experienced as damp or wet.

### Summary of the Invention

A fastening means for an absorbent article of the type discussed in the introduction has been achieved through the present invention as defined in claim 1, which significantly minimises the problems of previously-known fastening means. The invention is primarily characterised in that at least one belt portion comprising at least one nonwoven layer, which is a composite material comprising synthetic continuous filaments, e.g. spunlaid, and natural fibres, e.g. cellulose fibres. The natural fibres are suitably hydrophilic. Said layer is preferably hydroentangled. Here, and In the following, "entangled" means to tangle or wound together. Here, and in the following, "user" means the individual wearing the diaper.

The fastening means comprises a belt for a belt-diaper. To function as such a belt, the belt should exhibit a sufficiently high tensile strength, at the same time as being soft to the touch and having absorbing properties. A belt according to the invention is not just user-friendly as has just been described, but is also advantageously produced from a process technique point of view. In one embodiment of the invention, the belt can be at least partly formed of only one layer. This means that lamination of many materials as previously required to provide a belt, is not required. The belt is also relatively cheap to produce in comparison to other previously-known laminate belts, without compromising on qualities such as softness and strength.

Preferably, said nonwoven layer of composite material forms the user-facing side of the belt. According to the invention, at least one portion of the belt is only formed of said nonwoven layer of composite material.

The synthetic continuous filaments may be formed from, or alternatively comprise polypropene, polyethene or polylactide, or other suitable polymers.

The nonwoven layer of composite material according to the invention may further comprise synthetic staple fibres. The staple fibres exhibit a length of 1-15mm, preferably 3-7mm, and a thickness of circa 2-20 dtex. Long staple fibres help make the belt stiffer, while shorter staple fibres give an increased feel of textile material, which can be desirably in certain circumstances. The staple fibres can also be coloured to give the belt a colour other than that of the absorbent article.

Examples of suitable materials according to the invention are described in international patent application PCT/SE2004/001519 and in the Swedish patent application SE 0302874-3. According to the invention, the nonwoven layer of the belt comprises:
- 10-50% synthetic continuous filaments, preferably 15-35% synthetic continuous filaments
- 20-90% natural fibres, preferably 40-75% natural fibres,
- 0-50% synthetic staple fibres, preferably 5-25% synthetic staple fibres in weight percent based on the total weight of said nonwoven layer.

Of course, the various fibre contents cannot be selected such that the total amount exceeds 100%. In one embodiment, the fibre content is chosen such that the total amount of natural fibre and synthetic continuous filament in the belt's nonwoven layer exceeds 60%, preferably 70%, more preferably 80% and most preferably 90% in weight percent based on the total weight of said nonwoven layer of composite material.

When the nonwoven layer comprises 10% continuous filaments and 90% natural fibres, the belt gives high absorption capacity due to the high concentration of natural fibres. This can be advantageous on users who sweat a lot, such as for example upon use of the diaper in warm environments for longer periods. The belt gives a drier sensation against the skin as it sucks in the dampness which accumulates. However, a belt according to this embodiment is not as strong as an embodiment with a higher content of synthetic continuous filaments. 10% synthetic continuous filaments should therefore be regarded as the minimum limit. With higher amounts of synthetic continuous filaments, the nonwoven layer becomes more expensive. To maintain a strong yet cheap nonwoven layer of composite material, it is therefore advantageous to keep within the range 10-50%, preferably 15-35%, synthetic continuous filaments in weight percent based on the total weight of said nonwoven layer.

In another embodiment, in which the nonwoven layer comprises 50% continuous filaments and 50% hydrophilic fibres, a very strong nonwoven layer is obtained, which still shows absorbing properties. According to one embodiment of the invention, the belt comprises 10-50% synthetic staple fibres together with other fibres in the above-mentioned content ranges. The synthetic continuous filaments can for example exhibit a basis weight of 20-50 g/m². For the nonwoven layer of composite material, the basis weight is 50-150 g/m², preferably 70-120 g/m².

The belt is preferably breathable. By "breathable", it is meant that water vapour and other cases can pass through the belt.

### Short description of the Figures

In the following, the invention shall be more closely described, with reference to the following figures.

Hereby:
Fig. 1 shows a belt diaper seen towards the liquid permeable topsheet
Fig. 2 shows a belt portion connected to an absorbent article.

### Description of embodiments

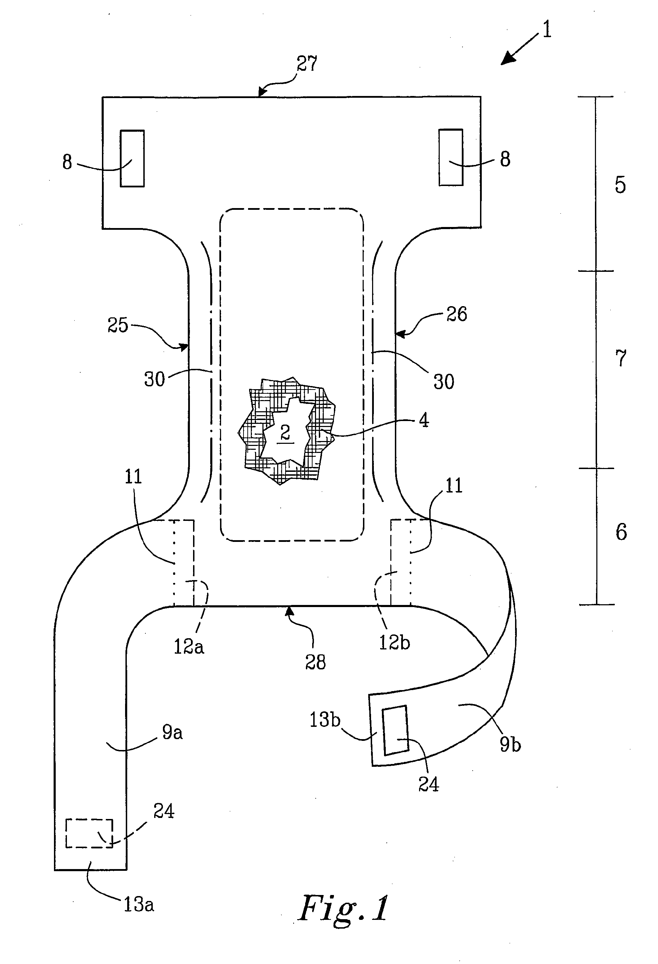

Figure 1 shows an embodiment of an absorbent article in the form of a belt diaper 1 comprising a backsheet material 2 which should preferably be liquid impermeable, a liquid permeable topsheet 3 and an absorbent member 4 enclosed therebetween. The liquid permeable topsheet 3 can comprise nonwoven material, bonded carded fibre material, perforated plastic film or combinations thereof. In the case there the backsheet material is liquid impermeable, it may comprise material such as plastic film, nonwoven material which is coated with a liquid-blocking material or a hydrophobic nonwoven which resists liquid penetration. The backsheet material 2 is preferably breathable. By "breathable material" is meant a material which has the ability to allow water vapour and other gases through. Examples of such materials are well known in the field and will not be described further here.

The backsheet material 2 and the topsheet 3 have a somewhat larger planar extension than the absorbent member 4, and extend outside the edges of the absorbent member. The backsheet material 2 and the topsheet 3 are mutually connected within the projecting edges, e.g. though gluing or welding with heat or ultrasound. On each side of, and substantially parallel with, the absorbent member 4, run elastic elements 30. So-called "leakage barriers" (not shown) may also be fixed in the longitudinal direction of the absorbent member 4.

The absorbent member 4 can be of any conventional kind. Examples of normally occurring absorbent materials are cellulose fluff pulp, tissue layers, highly absorbing polymers (so-called "superabsorbents" or SAP), absorbent foam material, absorbent nonwoven material and the like. It is usual to combine cellulose fluff pulp with superabsorbents in an absorbent member. Absorbent members built up of layers of different material with different properties in respect of liquid receiving ability, liquid distribution ability and storage ability are also common. These are known to the person skilled in the art, and do not therefore need to be described in detail. The thin absorbent cores which are normal in e.g. baby diapers and incontinence shields often comprise a compressed, mixed or layered structure of cellulose fluff pulp and superabsorbents.

The belt diaper is intended to encompass the lower portion of the wearer's trunk like a pair of absorbent pants. It has a front part 5 which in use is intended to be turned forward on the wearer and a rear portion 6 which in use is intended to be turned backwards on the user. A groin portion 7 is located between the front and rear portions, which is use is intended to be placed in the wearer's groin, between their legs. The front portion 5 possesses a pair of front fastening members 8 of the hook and loop type. Adhesive fastening agents can also be used.

The belt 9 comprises a first and a second belt portion 9a,9b. The belt portions are joined via a first end 12a, 12b along line 11 to the rear portion 6 of the diaper, for example by gluing or ultrasound welds. According to another embodiment, the belt portions can be formed from the same layer as the rear portion 6. The first and second belt portions 9a,9b are intended to be joined together through their other ends 13a,13b with a belt fastening member 24. The belt fastening member 24 can be formed from hook and loop type material, e.g. VELCRO®, or from an adhesive fastening agent. The front fastening member 8 of the front portion 5 is intended to be joined to the outside of the belt portions 9a,9b, in order to fasten the belt diaper to the desired pant-like form.

According to an alternative embodiment (not shown), the first and the second belt portion 9a,9b are fastened to the diaper's front portion 5 and are thereby intended to be joined together on the wearer's back. The fastening member 8 is thus arranged on the rear portion 6 of the diaper as a rear fastening member.

In an embodiment which is not shown, the backsheet 2 can be used as the front or rear fastening member 8.

To provide a comfortable fit, the belt portions 9a,9b should have a width of 5-20cm, preferably 7-15cm. The belt portions 9a,9b can be formed from a nonwoven layer of composite material which comprises synthetic continuous filaments, e.g. spunlaid, and natural fibres such as cellulose fibres. Suitably, said layer is hydroentangled. The nonwoven layer of composite material can also be a part of a laminate, i.e. a support material, which can form at least a part of the belt. When the nonwoven layer of composite material forms a part of a laminate, the laminate can preferably comprise a nonwoven material which is formed from a carded, thermobonded material such as e.g. polypropylene, polyester or bi-component fibres. Such a layer can act as loop material for a hook and loop type fastening system.

A preferred embodiment is that where the nonwoven layer of composite material is not completely encompassed by the laminate, but only 50% or less of the area of the nonwoven layer is covered by another material, e.g. a carded, thermobonded material.

The nonwoven layer of composite material can be adapted to act as a reception surface for the front fastening member 8. In the case where the front fastening members are comprised of adhesive tape flaps, the reception zone is in the form of a suitable plastic film. In case other types of fastening members are used instead of adhesive tape flaps, e.g. hook and loop types, other types of material are appropriate which can function as a reception surface for the fastening member in question. Elastic laminates are also suitable for use as material in the belt portions 9a,9b. An important detail is that the belt portions 9a,9b are preferably made breathable so as not to occlude (i.e. obstruct) the skin of the user wearing it.

Diaper 1 also shows two transverse side edges 27,28 as well as two longitudinal side edges 25, 26.

As shown in Fig. 1, the belt 9 in the belt-diaper can be formed from two belt portions 9a,9b, but in accordance with the invention, the belt 9 can also be formed from only one belt portion.

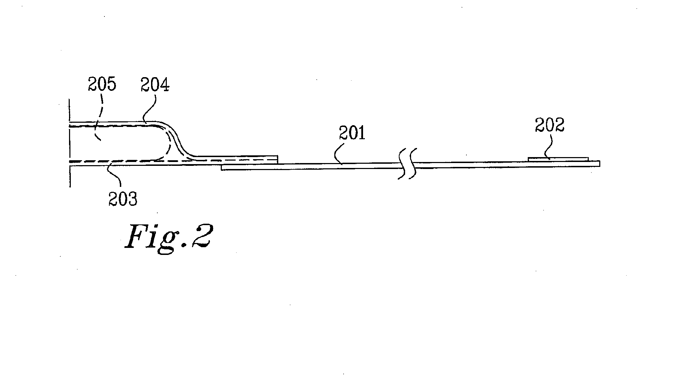

A belt portion 201 is shown in Figure 2 which alone can form a belt, or can be used together with a corresponding belt portion, see Fig. 1. In use, the belt is fastened about the user's torso. The belt portion 201 has a means for fastening 202 in the form of a surface provided with a hook material which is arranged on belt portion 201. Belt portion 201 is fixedly arranged on a backsheet 203. A absorbent body is placed between the backsheet 203 and the liquid permeable topsheet 204.

The belt portion 201 in Fig. 2 is made only of a composite nonwoven layer comprising 40% continuous filaments together with 60% cellulose fibres in weight percent based on the total weight of said nonwoven layer. The continuous filaments give tensile strength to the belt, while the cellulose fibres provide the belt with softness and the ability to absorb liquid. The amounts in the belt can be suited to give the belt varied properties within the scope of the invention.

### Manufacture and composition of the composite material

Filaments are fibres which are relatively long in proportion to their diameter; in principle they can in certain circumstances considered infinitely long relative to their diameter. They are produced through melting and extruding a thermoplastic polymer through small holes. The polymer is then cooled down, preferably by air being blown onto, and in line with the polymer threads. The polymer threads solidify, and can then be treated through being stretched, curled, crumpled or similar. Chemicals for surface treatment of the filaments can be applied to provide further properties of the filaments.

The filaments can also be manufactured through chemical reactions, via a solution with fibre-forming reagents which are placed in a reagent, e.g. through spinning viscose fibres from cellulose xanthate solution in sulphuric acid.

So-called meltblown fibres are produced via extruding melted thermoplastic polymers through small holes so that fine threads a formed, at the same time as converging air streams are directed forwards the polymer threads. The polymer threads are drawn out to long continuous filaments with a small diameter. Manufacture of meltblown fibres is described in detail in US 3,849,241 and US 4,048,364. The fibres can be so-called microfibres or macrofibres depending on their dimensions. Microfibres usually have a diameter of ≤20 µm, usually 2-12 µm. Macrofibres have a diameter of over 20 µm, usually 21-100 µm.

So-called spunbond filaments are produced in a like manner as that described above, but the air streams have a lower temperature and the air streams stretch the filament to achieve a suitable diameter. The fibre diameter is usually over 10 µm, usually 10-110 µm. Production of spunbond is described for example in patent document US 4,812,864 and US 5,545,371.

As a group, spunbond and meltblown filaments are usually called "spun laid filaments". This involves that they are laid directly on a moveable surface to make a layer which is bound together later in the process. In this way, the synthetic continuous filaments have a diameter of 2-100 µm, preferably 10-100 µm.

Thermoplastic polymers which have sufficiently coherent strength properties which allow them to be stretched out in their melted condition, as described above, can in principle be used to produce meltblown or spunbond filament. Examples of polymers which can be used are: polyolefins, such as polyethene, polypropene, polyamide, polyester and polylactides. Copolymers of these polymers may also be used.

Many different natural fibres can be found which may be used in accordance with the invention. Fibres which have the capacity to absorb water and tend to help the formation of a cohesive layer are especially useful. The natural fibres are preferably hydrophilic. Examples of natural fibres are cellulose fibres, cotton fibres, hemp fibres, leaf fibres, jute fibres and the like. Preferred fibres are paper pulp fibres. Pulp fibres are preferably between 1-5mm. Recycled pulp fibres may be used. Examples of paper pulp fibres are chemical pulps, CTMP and TMP.

Staple fibres can be produced with the same material and same processes as mentioned earlier. Staple fibres can be treated with so-called "spin finish", i.e. surface treated and curled or crumpled. Spin finishing is usually used to give the fibres a hydrophilic surface when they are used in e.g. the outer surface of diapers. As opposed to filaments, staple fibres are cut to suitable lengths. The lengths can vary depending on the purpose. When the staple fibres are used in hydroentangled nonwoven layers, the length is 1-15mm, preferably 3-7mm.

One way to produce a material according to the invention comprises the following steps:
First a continuous material web is provided, upon which the continuous filaments can be laid down. Excess air is sucked through said material web so that a layer of filament is formed on the material web. The layer of continuous filaments is then moved to a wet-laying section, where a slurry comprising a mixture of natural fibres and water, possibly together with staple fibres as well, is wet-laid upon the layer of continuous filaments. Remaining water is sucked away through the material web. In one embodiment, the slurry containing a mixture of natural fibres and water, possibly together with staple fibres, is wet-laid on the layer of continuous filaments so that they at least partly penetrate into the layer of continuous filaments.

The layer of continuous filaments and the mixture of natural fibres are transported to an apparatus for hydroentangling. Here, the filaments and fibres and mixed, and bound together to a nonwoven layer by many small water jet streams being sprayed on the layer at high pressure.

The hydroentangled layer, i.e. the nonwoven layer, is transported to a drying stage where the layer is dried. The layer may be transported further for rolling, cutting and packing.

In accordance with the previously-described method, the continuous filaments are laid down directly on the continuous material web, where they form an unbonded layer. This unbonded layer can in this instance look like a net structure. By letting the filaments cool down sufficiently before they land on the material web, the layer keeps a certain amount of mobility. This is because, after cooling down, the filaments are not sticky. The speed at which the filaments are laid down on the material web is much higher than the speed of the material web. This means that the filaments form irregular loops and bends as they are laid on the material web. This contributes greatly to the irregularity that the filaments make in the layer. The basis weight of the layer formed with continuous filaments should be between 2-50 g/m².

Pulp fibres, and possibly also staple fibres, which can be added as required, are carefully blended to a slurry. Possible additives, such as wet or dry strength substances, retention agents, dispersing agents, pigment or mixtures thereof can be added. The mixture is pumped out via a headbox on the material web, where it then lies on the unbonded continuous filaments. Pulp fibres and staple fibres will mainly stay above the layer of continuous filaments. Only a few fibres will penetrate between the filaments. The excess water is sucked through the material web and the fiber layer with the help of a drying box which is placed under the material web.

The fibre web of continuous filament, pulp fibre and possibly staple fibre is hydroentangled while it still lies on the material web. Hydroentangling mixes the fibres intensively and binds the fibres together to a nonwoven of composite material. A full description of hydroentangling is described in patent CA 841 938 A.

In hydroentangling, the various fibres will become significantly homogeneously mixed and integrated with each other, and form a nonwoven of composite material. The finer mobile spunlaid filaments twist themselves together and build a strong material together with the other fibres. The energy which is required for entangling is relatively low, i.e. material of this type is easy to entangle. The energy which is put in to such an entangling is in the order of mangnitude 50-500kwh/ton.

In one embodiment, no binding - e.g. thermobonding or hydroentangling - of the continuous filaments is carried out before pulp fibres and possibly staple fibres are laid on the continuous filaments. The continuous filaments should preferably be free to move, which makes mixing, integration and binding together of the fibres easier under hydroentangling. With this step, possible thermobonded points would prevent pulp fibres and staple fibres from being caught up or entangled in the continuous filaments, as they would be immobilised by the bonding points. A two-layer material will be produced instead of a well-mixed and integrated composite material.

The strength of a hydroentangled material based on pulp fibres or staple fibres or mixtures thereof depends mostly upon the number of bonding points, i.e. points where the fibres are entangled together. Long fibres are therefore preferable. When a filament is used, the strength of the material comes primarily from the filaments. The strength is also obtained relatively quickly upon hydroentangling. Therefore, the energy in the entangling process in used primarily for mixing the fibres and to maintain good integration of the fibres. The unbonded, open structure which the filaments have makes this integration easier.

The nonwoven layer of composite material is then dried through a conventional drying step.

## Claims

1. A belt (9) for an absorbent article (1), said belt (9) comprising at least one belt portion (9a, 9b, 201), wherein at least one belt portions (9a, 9b, 201) is/are attached to the rear portion (6), alternatively the front portion (5) of the absorbent article (1) and is/are intended to be first joined about the waist of a wearer, and then the other of the front portion (5), alternatively the rear portion (6), is intended to be joined to the outside of the belt portion(s) (9a, 9b, 201), wherein said belt portions (9a, 9b, 201) comprise at least one nonwoven layer,
***characterised in that***
at least one nonwoven layer in the belt portion (9a, 9b, 201) is a composite material which comprises synthetic continuous filaments and natural fibres.

2. Belt (9) for an absorbent article according to claim 1, ***characterised in that*** the natural fibres are hydrophilic fibres.

3. Belt (9) for an absorbent article according to claim 2, ***characterised in that*** the hydrophilic fibres are cellulose fibres.

4. Belt (9) for an absorbent article according to any of claims 1-3, ***characterised in that*** the synthetic continuous fibres are spunlaid.

5. Belt (9) for an absorbent article according to any of the preceding claims, ***characterised in that*** said nonwoven layer of composite material is hydroentangled.

6. Belt (9) for an absorbent article according to any of the preceding claims, ***characterised in that*** the synthetic continuous filaments comprise polypropene, polyethene or polylactide.

7. Belt (9) for an absorbent article according to any of the preceding claims, ***characterised in that*** said nonwoven layer of composite material further comprises synthetic staple fibres with a length of 1-15mm.

8. Belt (9) for an absorbent article according to any of claims 1-6, ***characterised in that*** said nonwoven layer of composite material further comprises synthetic staple fibres with a length of 3-7mm

9. Belt (9) for an absorbent article according to any of the preceding claims, ***characterised in that*** said nonwoven layer of composite material comprises:
10-50% synthetic continuous filaments
20-90% natural fibres
0-50% synthetic staple fibres, in weight percent based on the total weight of said nonwoven layer.

10. Belt (9) for an absorbent article according to any of the preceding claims, *characterisad in that* said nonwoven layer of composite material has a basis weight of 50-150 g/m².

11. Belt (9) for an absorbent article according to any of claims 1-9, ***characterised in that*** said nonwoven layer of composite material has a basis weight of 70-120 g/m²

12. Belt (9) for an absorbent article according to any of the preceding claims, ***characterised in that*** said nonwoven layer of composite material forms the user-facing side of the belt.

13. Belt (9) for an absorbent article according to any of the preceding claims, ***characterised in that*** at least one portion of the belt is only formed of said nonwoven layer of composite material.

14. Belt (9) for an absorbent article according to any of the preceding claims, ***characterised in that*** the total amount of natural fibre and synthetic continuous filament in the nonwoven layer of the belt exceeds 60% in weight percent based on the total weight of said nonwoven layer of composite material.

15. Belt (9) for an absorbent article according to any claims 1-13, ***characterised in that*** the total amount of natural fibre and synthetic continuous filament in the nonwoven layer of the belt exceeds 70% in weight percent based on the total weight of said nonwoven layer of composite material.

16. Belt (9) for an absorbent article according to any of claims 1-13, ***characterised in that*** the total amount of natural fibre and synthetic continuous filament in the nonwoven layer of the belt exceeds 80% in weight percent based on the total weight of said nonwoven layer of composite material.

17. Belt (9) for an absorbent article according to any of claims 1-13, ***characterised in that*** the total amount of natural fibre and synthetic continuous filament in the nonwoven layer of the belt exceeds 90% in weight percent based on the total weight of said nonwoven layer of composite material.

## Patentansprüche

1. Gürtel (9) für einen saugfähigen Artikel (1), der Gürtel (9) mit mindestens einem Gürtelabschnitt (9a, 9b, 201), wobei mindestens ein Gürtelabschnitt (9a, 9b, 201) an dem hinteren Abschnitt (6), alternativ dem vorderen Abschnitt (5), des saugfähigen Artikels (1) angebracht ist/sind und vorgesehen ist/sind, um zuerst um die Taille eines Trägers verbunden zu werden, und dann der andere vordere Abschnitt (5), alternativ der hintere Abschnitt (6), vorgesehen ist, mit der Außenseite des Gürtelabschnitts/der Gürtelabschnitte (9a, 9b, 201) verbunden zu werden, wobei die Gürtelabschnitte (9a, 9b, 201) mindestens eine Vlieslage aufweisen,
**dadurch gekennzeichnet, dass**
mindestens eine Vlieslage in dem Gürtelabschnitt (9a, 9b, 201) ein Verbundmaterial ist, dass synthetische ununterbrochene Filamente und Naturfasern aufweist.

2. Gürtel (9) für einen saugfähigen Artikel nach Anspruch 1, **dadurch gekennzeichnet, dass** die Naturfasern hydrophile Fasern sind.

3. Gürtel (9) für einen saugfähigen Artikel nach Anspruch 2, **dadurch gekennzeichnet, dass** die hydrophilen Fasern Zellulosefasern sind.

4. Gürtel (9) für einen saugfähigen Artikel nach einem der Ansprüche 1-3, **dadurch gekennzeichnet, dass** die synthetischen ununterbrochenen Fasern spinngelegte Vliese sind.

5. Gürtel (9) für einen saugfähigen Artikel nach einem der vorstehenden Ansprüche, **dadurch gekennzeichnet, dass** die Vlieslage des Verbundmaterials wasserverfilzt ist.

6. Gürtel (9) für einen saugfähigen Artikel nach einem der vorstehenden Ansprüche, **dadurch gekennzeichnet, dass** die synthetischen ununterbrochenen Filamente Polypropen, Polyethylen oder Polylactid aufweisen.

7. Gürtel (9) für einen saugfähigen Artikel nach einem der vorstehenden Ansprüche, **dadurch gekennzeichnet, dass** die Vlieslage des Verbundmaterials ferner synthetische Stapelfasern mit einer Länge von 1-15 mm aufweist.

8. Gürtel (9) für einen saugfähigen Artikel nach einem der Ansprüche 1-6, **dadurch gekennzeichnet, dass** die Vlieslage des Verbundmaterials ferner synthetische Stapelfasern mit einer Länge von 3-7 mm aufweist.

9. Gürtel (9) für einen saugfähigen Artikel nach einem der vorstehenden Ansprüche, **dadurch gekennzeichnet, dass** die Vlieslage des Verbundmaterials aufweist:
10-50 % synthetische ununterbrochene Filamente 20-90 % Naturfasern
0-50 % synthetische Stapelfasern, und zwar in Gewichtsprozent basierend auf dem Gesamtgewicht der Vlieslage.

10. Gürtel (9) für einen saugfähigen Artikel nach einem der vorstehenden Ansprüche, **dadurch gekennzeichnet, dass** die Vlieslage des Verbundmaterials ein Flächengewicht von 50-150 g/m² aufweist.

11. Gürtel (9) für einen saugfähigen Artikel nach einem der Ansprüche 1-9, **dadurch gekennzeichnet, dass** die Vlieslage des Verbundmaterials ein Flächengewicht von 70-120 g/m² aufweist.

12. Gürtel (9) für einen saugfähigen Artikel nach einem der vorstehenden Ansprüche, **dadurch gekennzeichnet, dass** die Vlieslage des Verbundmaterials die dem Benutzer zugewandte Seite des Gürtels ausbildet.

13. Gürtel (9) für einen saugfähigen Artikel nach einem der vorstehenden Ansprüche, **dadurch gekennzeichnet, dass** mindestens ein Abschnitt des Gürtels nur aus der Vlieslage des Verbundmaterials ausgebildet ist.

14. Gürtel (9) für einen saugfähigen Artikel nach einem der vorstehenden Ansprüche, **dadurch gekennzeichnet, dass** die Gesamtmenge an Naturfasern und synthetischen ununterbrochenen Filamenten in der Vlieslage des Gürtels basierend auf dem Gesamtgewicht der Vlieslage des Verbundmaterials 60 Gewichtsprozent übersteigt.

15. Gürtel (9) für einen saugfähigen Artikel nach einem der Ansprüche 1-13, **dadurch gekennzeichnet, dass** die Gesamtmenge an Naturfasern und synthetischen ununterbrochenen Filamenten in der Vlieslage des Gürtels basierend auf dem Gesamtgewicht der Vlieslage des Verbundmaterials 70 Gewichtsprozent übersteigt.

16. Gürtel (9) für einen saugfähigen Artikel nach einem der Ansprüche 1-13, **dadurch gekennzeichnet, dass** die Gesamtmenge an Naturfasern und synthetischen ununterbrochenen Filamenten in der Vlieslage des Gürtels basierend auf dem Gesamtgewicht der Vlieslage des Verbundmaterials 80 Gewichtsprozent übersteigt.

17. Gürtel (9) für einen saugfähigen Artikel nach einem der Ansprüche 1-13, **dadurch gekennzeichnet, dass** die Gesamtmenge an Naturfasern und synthetischen ununterbrochenen Filamenten in der Vlieslage des Gürtels basierend auf dem Gesamtgewicht der Vlieslage des Verbundmaterials 90 Gewichtsprozent übersteigt.

## Revendications

1. Ceinture (9) destinée à un article absorbant (1), ladite ceinture (9) étant constituée d'au moins une partie ceinture (9a, 9b, 201), au moins une des parties ceinture (9a, 9b, 201) étant fixée à la partie arrière (6), en variante à la partie avant (5) de l'article absorbant (1), et étant destinée à être d'abord passée autour de la taille d'un utilisateur, puis l'autre partie de l'article absorbant, c'est-à-dire la partie avant (5), en variante la partie arrière (6) étant destinée à être attachée sur l'extérieur de la partie ceinture (des parties ceinture) (9a, 9b, 201), lesdites parties ceinture (9a, 9b, 201) comprenant au moins une couche de non-tissé,
***caractérisée en ce qu'***
au moins une couche de non-tissé présente dans la partie ceinture (9a, 9b, 201) étant un matériau composite qui comprend des filaments synthétiques continus et des fibres naturelles.

2. Ceinture (9) destinée à un article absorbant selon la revendication 1 ***caractérisée en ce que*** les fibres naturelles sont des fibres hydrophiles.

3. Ceinture (9) destinée à un article absorbant selon la revendication 2 ***caractérisée en ce que*** les fibres hydrophiles sont des fibres cellulosiques.

4. Ceinture (9) destinée à un article absorbant selon l'une quelconque des revendications 1 à 3 ***caractérisée en ce que*** les fibres synthétiques continues sont déposées au filage.

5. Ceinture (9) destinée à un article absorbant selon l'une quelconque des revendications qui précèdent ***caractérisée en ce que*** ladite couche de non-tissé en matériau composite est enchevêtrée hydrauliquement.

6. Ceinture (9) destinée à un article absorbant selon l'une quelconque des revendications qui précèdent ***caractérisée en ce que*** les filaments synthétiques continus comprennent le polypropylène, le polyéthylène ou le polylactide.

7. Ceinture (9) destinée à un article absorbant selon l'une quelconque des revendications qui précèdent ***caractérisée en ce que*** ladite couche de non-tissé en matériau composite comprend en outre des fibres synthétiques coupées dont la longueur va de 1 à 15 mm.

8. Ceinture (9) destinée à un article absorbant selon l'une quelconque des revendications 1 à 6 ***caractérisée en ce que*** ladite couche de non-tissé en matériau composite comprend en outre des fibres synthétiques coupées dont la longueur va de 3 à 7 mm.

9. Ceinture (9) destinée à un article absorbant selon l'une quelconque des revendications qui précèdent ***caractérise en ce que*** ladite couche de non-tissé en matériau composite comprend, en poids par rapport au poids total de ladite couche de non-tissé
de 10 à 50 % de filaments synthétiques continus
de 20 à 90 % de fibres naturelles
de 0 à 50 % de fibres synthétiques coupées.

10. Ceinture (9) destinée à un article absorbant selon l'une quelconque des revendications qui précèdent ***caractérisée en ce que*** ladite couche de non-tissé en matériau composite a une masse surfacique allant de 50 à 150 g/m².

11. Ceinture (9) destinée à un article absorbant selon l'une quelconque des revendications 1 à 9 ***caractérisée en ce que*** ladite couche de non-tissé en matériau composite a une masse surfacique allant de 70 à 120 g/m².

12. Ceinture (9) destinée à un article absorbant selon l'une quelconque des revendications qui précèdent ***caractérisée en ce que*** ladite couche de non-tissé en matériau composite constitue le côté de la ceinture orienté vers l'utilisateur.

13. Ceinture (9) destinée à un article absorbant selon l'une quelconque des revendications qui précèdent ***caractérisée en ce qu'**au* moins une partie de la ceinture n'est constituée que de ladite couche de non-tissé en matériau composite.

14. Ceinture (9) destinée à un article absorbant selon l'une quelconque des revendications qui précèdent ***caractérisée en ce que*** la quantité totale de fibres naturelles et de filaments synthétiques continus présents dans la couche de non-tissé de la ceinture est supérieure à 60 % en poids par rapport au poids total de ladite couche de non-tissé en matériau composite.

15. Ceinture (9) destinée à un article absorbant selon l'une quelconque des revendications 1 à 13 ***caractérisée en ce que*** la quantité totale de fibres naturelles et de filaments synthétiques continus présents dans la couche de non-tissé de la ceinture est supérieure à 70 % en poids par rapport au poids total de ladite couche de non-tissé en matériau composite.

16. Ceinture (9) destinée à un article absorbant selon l'une quelconque des revendications 1 à 13 ***caractérisée en ce que*** la quantité totale de fibres naturelles et de filaments synthétiques continus présents dans la couche de non-tissé de la ceinture est supérieure à 80 % en poids par rapport au poids total de ladite couche de non-tissé en matériau composite.

17. Ceinture (9) destinée à un article absorbant selon l'une quelconque des revendications 1 à 13 ***caractérisée en ce que*** la quantité totale de fibres naturelles et de filaments synthétiques continus présents dans la couche de non-tissé de la ceinture est supérieure à 90 % en poids par rapport au poids total de ladite couche de non-tissé en matériau composite.
